Europäisches Patentamt

European Patent Office

Office européen des brevets

(11) Publication number : **0 512 841 A1**

(12) # EUROPEAN PATENT APPLICATION

(21) Application number : **92304137.0**

(22) Date of filing : **08.05.92**

(51) Int. Cl.⁵ : **A61L 9/14**

(30) Priority : **08.05.91 IE 1568/91**

(43) Date of publication of application :
**11.11.92 Bulletin 92/46**

(84) Designated Contracting States :
**AT BE CH DE DK ES FR GB GR IT LI LU MC NL
PT SE**

(71) Applicant : **MANROSE HOLDINGS LIMITED**
**Merchants House**
**Merchants Quay, Dublin 8 (IE)**

(72) Inventor : **Flood, Christopher**
**24 Drapier Green**
**Wadelai Estate, Dublin 9 (IE)**

(74) Representative : **Barlow, Roy James et al**
**J.A. KEMP & CO. 14, South Square, Gray's Inn**
**London WC1R 5LX (GB)**

(54) **Air freshener device.**

(57)    An air freshener device primarily for use with
a flush toilet in which actuation of the flushing
lever actuates the air freshener device to re-
lease a charge of deodorant product.

Fig.1.

This invention relates to an air freshener device. In particular, it relates to an air freshener device which will dispense a metered amount of perfume on demand.

An air freshener device which dispenses perfume at predetermined intervals is already known. Such a device incorporates a timer mechanism which requires some form of motive power for its operation. The known device is thus relatively complicated from the point of view of manufacture, installation and maintenance. Furthermore, the prior air freshener device can be uneconomical in that perfume may be dispensed at times when it is not required.

It is therefore an object of the present invention to provide an air freshener device which is easy to manufacture and maintain and is economical in use.

According to the invention there is provided an air freshener device which comprises means for mounting or releasably holding a container having air freshener product therein the container having a product release means-having an aperture associated -therewith for release of the product and movable between a first position of use and a second position and vice versa; a pivotable lever means contactable with said product release means; an actuation means connected to the lever means for causing the lever means to move from a primary position of use to a secondary position of use and vice versa; the arrangement being such that when the actuation means moves the lever means from the primary position of use to the secondary position of use, the product release means is moved from the first position of use to the secondary position of use thereby releasing a charge of the product.

Essentially, the air freshener device of the invention is based on a spring lever action, releasing a perfumed spray from a replaceable container and requires no secondary motive power.

The invention will be understood in greater detail from the following description of preferred embodiments thereof given by way of example only and with reference to the accompanying drawings in which:

Figure 1 is a rear sectional elevation of an air freshener device according to a first embodiment of the invention;

Figure 1A is an enlargement of part of the device of Figure 1;

Figure 1B is a plan view of a decorative diffuser for use with the device of Figure 1;

Figure 2 is a front elevation of an air freshener device according to a second embodiment of the invention;

Figure 3 is a perspective view of an air freshener device according to a third embodiment of the invention;

Figure 4 is a rear sectional elevation of an air freshener device according to a fourth embodiment of the invention; and

Figure 5 is an exploded view of part of the air freshener device of Figure 4 of the drawings.

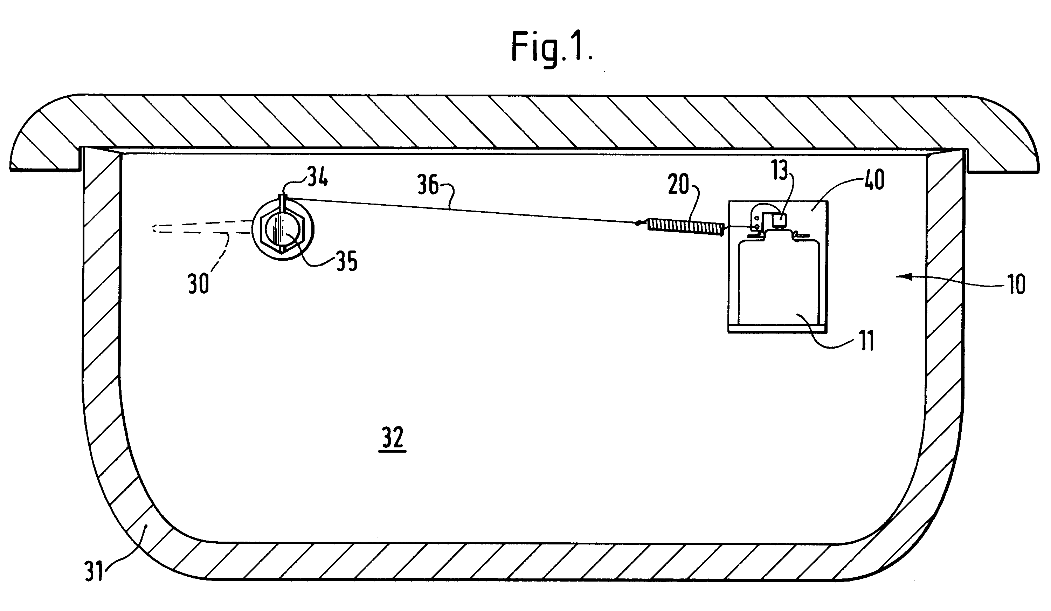

Referring now to the drawings, and in particular to Figures 1, 1a and 1b thereof, there is shown an air freshener device 10 according to the invention. The air freshener device 10 comprises a pivotable lever 15 which is L-shaped in the embodiment shown, a casing 40, and a spring loaded lever actuation means in the form of a coiled spring 20. The casing 40 is mounted on an interior surface 32 of the front wall 33 of a cistern 31 associated with a toilet (not shown). The container 11 having air freshener product therein is releasably held in the casing 40 by means of clips 43. Additional support is provided by a lip 42 at the lower end of the casing 40.

The container 11 comprises a spring loaded plunger 12 which has a head 13. The head 13 is rotatable and has a nozzle 14. The lever 15 which is held in the casing 40 by a retaining pin 41 has a leg 16 arranged so that it is in contact with the plunger head 13. The other leg 17 of the lever 15 has an opening 18 to which is connected the coiled spring 20 which in turn is connected by means of a wire 36 to a pin 34 associated with the spindle of a handle 30 of the toilet cistern 31.

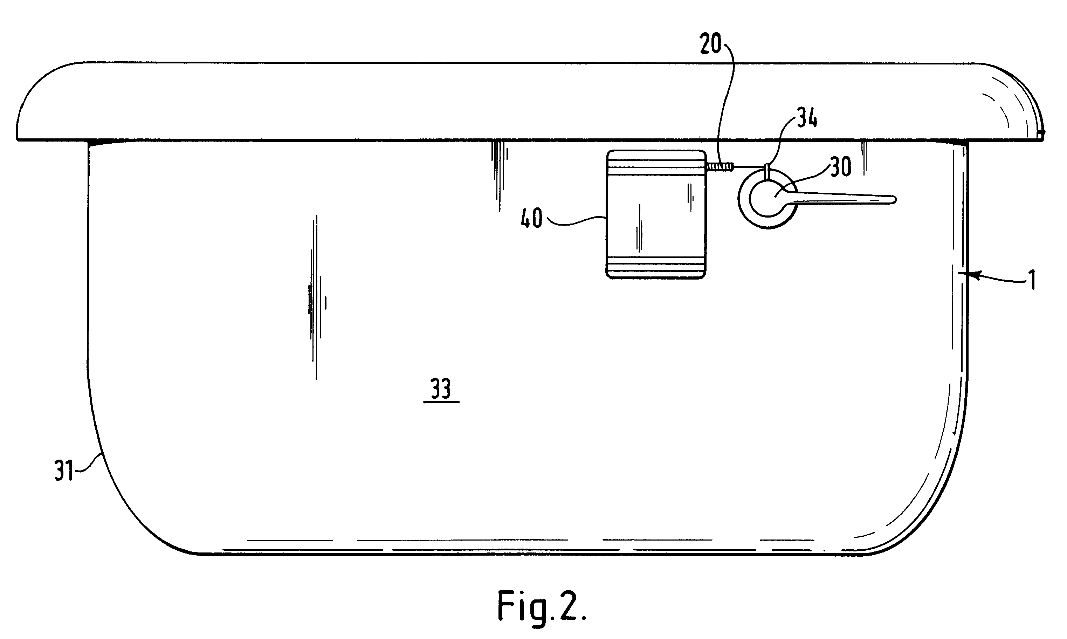

The casing 40 has an aperture (not shown) which is in alignment with the nozzle 14 which in turn is in alignment with an opening (not shown) of the cistern 31. The majority of modern cisterns 31 comprise an opening on the right and left of the front wall 33 thereof so that the handle 30 used for flushing the toilet may be fitted in either opening. The opening not used for the handle 30 is normally fitted with a plug. In the embodiments of Figures 1 and 2 of the drawings, the plug has been removed and replaced by a decorative diffuser 50 which has vents 51.

The device shown in Figure 1 operates as follows. When the toilet is flushed by movement of the handle 30 from a first position of use to a second position of use, the spindle 35 and the pin 34 rotate in an anticlockwise direction as viewed in Figure 1 of the drawings thereby pulling the wire 36 to the left and thus extending the spring 20. The extended spring 20 causes the lever 15 to rotate in a clockwise direction as viewed in Figure 1 and 1a of the drawings so that the leg 16 thereof presses on the plunger head 13 causing the plunger 12 to be depressed. The container 11 holds concentrated perfume (not shown) under pressure, so that the depression of the plunger 12 causes a measured spray charge of perfume to be dispensed through the nozzle 14. When the handle 30 returns to its first position of use, the plunger 12 also returns to its first position of use, resetting plunger, ready for next use.

The decorative diffuser 50 is provided on the front wall 33 of the cistern 31 for aesthetic reasons and to aid diffusion. It is suitably made of a plastics material. If desired, absorbent material may be placed between the decorative diffuser 50 and the opening (not shown) of the cistern 31 so that the perfume spray when dispensed may be absorbed by the absorbent material and then released slowly to the surrounding area over a period of time.

In Figure 2 of the drawings, the air freshener device 10 according to the invention is identical to that described in Figure 1 except that the device 10 is mounted on the exterior surface of front wall 33 of the cistern 31. The device 10 operates in the manner described for Figure 1. The casing 40 is suitably decorative and contains one or more outlets (not shown) through which the liquid perfume may be released. This arrangement is convenient for use on older type cisterns which may not have been provided with two openings on the front wall 33.

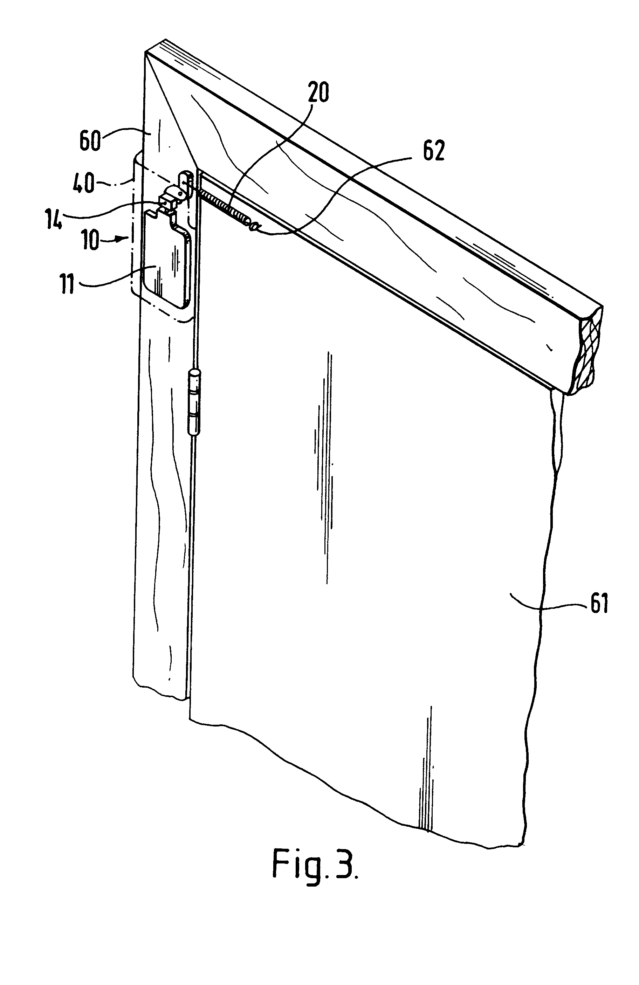

A third embodiment of the invention is shown in Figure 3 of the drawings. The air freshener device 10 is similar to that described in Figure 1. In this embodiment, the container 11 is held in the casing 40 which is mounted on a frame 60 of a door 61. One end of the coiled spring 20 is attached as in Figure 1 to the opening 18 of the leg 17 of the lever 15 and the other end of the coiled spring 20 is attached to the door 61 by means of a hook 62. This embodiment can be, either actuation on opening or actuation on closing.

The device of Figure 3 operates as follows. When the door 61 is opened or closed, the coiled spring 20 contracts and acts on the lever 15 which in turn acts on the plunger 12 causing a spray or charge of perfume to be dispensed through the nozzle 14 in the manner described for the embodiment of Figure 1. Actuation resets on the closing or opening of the door 61 which is advantageously a swing-type.

The casing 40 may be of any suitable material, metal or plastics being preferred.

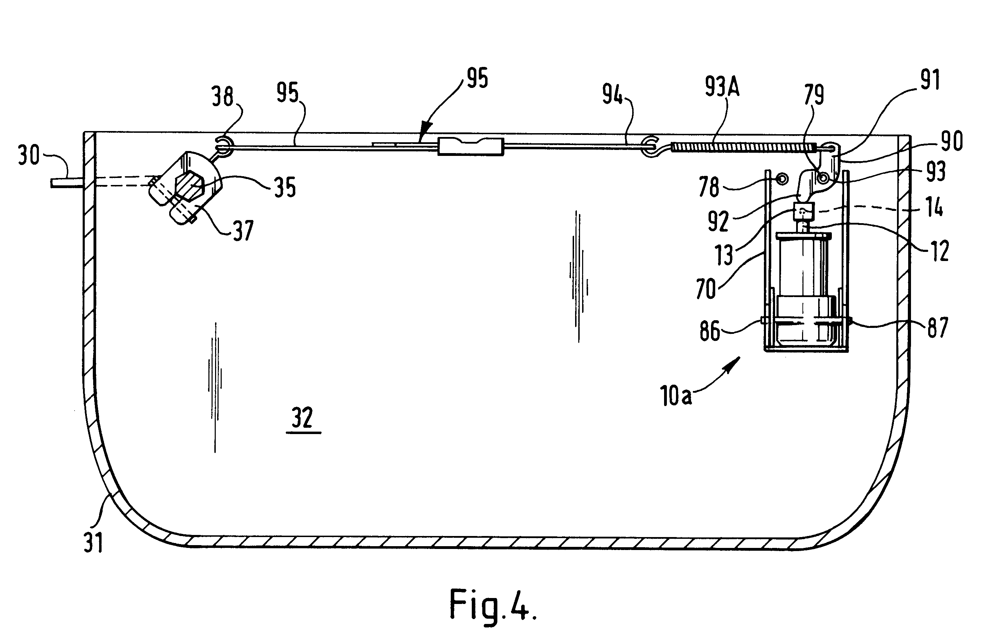

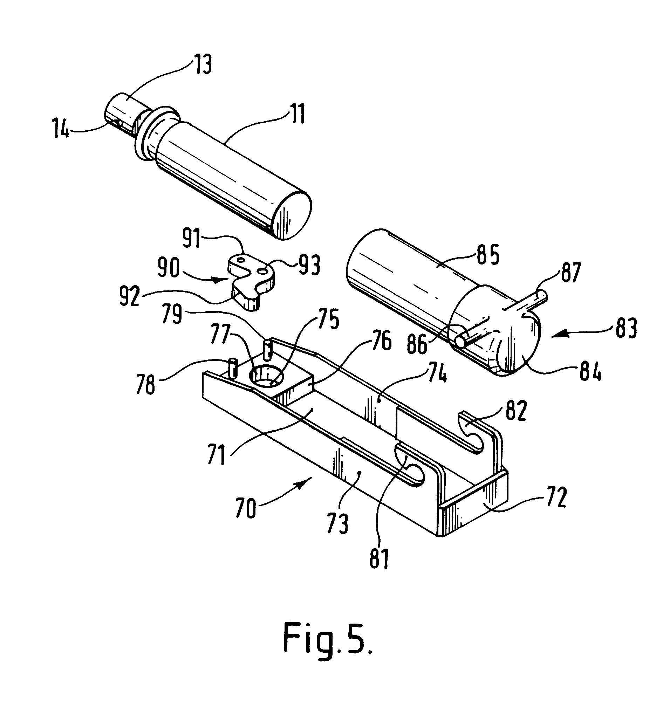

Referring now to Figures 4 and 5 of the drawings, there is shown a fourth embodiment of an air freshener device 10a according to the invention. As described with respect to Figure 1 of the drawings, there is shown the interior surface 32 of the front wall 33 of a toilet cistern 31. The front wall 33 has two openings (not shown). One of these openings has the spindle 35 of the handle 30 mounted for rotational movement therein, the spindle 35 having a first means (not shown) mounted thereon for enabling a rod (not shown) to be connected to the flush mechanism (not shown) of the toilet cistern 31. Rotation of the handle 30 causes the flush mechanism to operate. Also mounted on the spindle 35 is a second means 37 which is rotatable therewith. The second means 37 has a hook 38 attached thereto.

The other of these openings incorporates a mounting means (not shown) for enabling a casing 70 to be mounted on the interior surface 32 of the wall 33. The casing 70 comprises a front wall 71, a base 72 and two side walls 73, 74. The casing has an opening 75 which not only serves to enable the mounting means to be connected to the casing 70 but also provides an in register opening with the other opening of the front wall 33. Sandwiched between the walls 73, 74 is a mounting block 76 having an aperture 77 and two pins 78, 79 upstanding therefrom. The aperture 77 in register with the opening 75 of the casing.

Near the base 72, each of the side walls 73, 74 projects in a rearward direction relative to the front wall 71 to provide respective hook like members 81, 82.

A container housing 83 is provided having a base 84 and an open-mouthed tubular portion 85 integral therewith. The base 84 has a pair of pivot projections 86, 87 releasably engageable with the members 81, 82 so as to enable rotation of the housing 83 relative to the casing 70 about the axis formed by the pivot projections 86, 87.

A lever means 90 is provided having a first leg 91 and a second leg 92. The lever means 90 has an aperture 93 engageable with either of the pins 78, 79 being retained thereon by suitable means (not shown).

The leg 91 has attached thereto a spring 93 which in turn is connectable to one end 94 of an element 95 the other end 95 of the element 95 being connected to the hook 38.

To accommodate varying distances between the openings of the front wall 33, the element 95 may be of the type which is length adjustable and once adjusted for length cannot be unintentionally altered.

In use, the housing 83 is rotated so as to point the mouth upwardly and rearwardly relative to the casing 70. Into the housing 83 is inserted the container 11 having the spring loaded plunger 12 and the head 13 incorporating a nozzle 14 attached thereto. The housing 83 is rotated forwardly so that the leg 92 is in contact with the head 13.

The positions of the components as shown in Figure 4 of the drawings reflect the quiescent state of the toilet cistern i.e. when the flush mechanism is not being actuated. In that condition, no perfume is dispersed from the container 11.

Upon actuation of the handle 30 from the first position of use to the second position of use, the spindle 35 is rotated anti-clockwise (as viewed in Figure 4 of the drawings) thereby causing the hook 38 to move to the left. Movement of the hook 38 to the left causes a pulling action on the element 95 and the spring 93 thereby causing rotation of the lever means 90 about the pin 78 or 79 as the case may be. Thus, the legs 91, 92 rotate anti-clockwise the action of which causes the leg 92 to depress the plunger 12 thereby causing the release of a measured spray charge of perfume from the container 11. The nozzle 14 is positioned so as to cause the perfume to disperse through the in register aperture 77 and opening 75 resulting in perfume being dispersed into the atmosphere outside the cistern 31. On return of the handle 30 to the first position of use, the plunger 12 also returns to the first position of use thereby resetting the system, ready for next use. If desired, the decorative diffuser 50 may be of a similar type to that described with reference to Figure 1 of the drawings.

The container 11 for the perfume is readily replaced by a full container when required. The formula of the perfume contained in the cartridge is such that when a spray or charge thereof is released, unpleasant odours in the immediate environment are minimised. A typical container 11 is approximately 3.5 cm in length, 6 cm in height and 1.5 cm in width (Figure 1 of the drawings) or about 85 mm high (including plunger) and about 20 mm in diameter in the case of Figure 4 of the drawings. The container 11 contains concentrated perfume under pressure and has a metered spray. It is intended that the amount of perfume released will be sufficient to alleviate the effects of unpleasant odours. A typical container 11 releases at least 250 sprays. The amount of liquid in the container 11, depending on the strength of the perfume, is envisaged to be in the region of 8 ml (in the case of Figure 1 of the drawings) and 100 ml (in the case of Figure 4 of the drawings). Each metered spray will release approximately 0.032 ml.

The air freshener device 10 or 10a according to the invention has a number of advantages, including:

(1) Batteries are not required;

(2) Electric wirings or transformers are not required;

(3) The device is easy to manufacture and install;

(4) Little or no maintenance is required;

(5) The device operates only on demand, e.g. when a toilet is flushed or a door either opened or closed;

(6) Alternative fitting arrangements are possible;

(7) The device is suitable for use in large or small bathrooms or cubicles;

(8) The decorative casing or diffuser may be provided in a range of colours and various fragrances may be used.

**Claims**

1. An air freshener device which comprises means for mounting or releasably holding a container having air freshener product therein the container having a product release means having an aperture associated therewith for release of the product and movable between a first position of use and a second position and vice versa; a lever means contactable with said product release means; an actuation means connected to the lever means for causing the lever means to move from a primary position of use to a secondary position of use and vice versa; the arrangement being such that when the actuation means moves the lever means from the primary position of use to the secondary position of use, the product release means is moved from the first position of use to the secondary position of use thereby releasing a charge of the product.

2. A device as claimed in claim 1 wherein the actuation means comprises a door or a handle.

3. A device as claimed in claim 1 wherein the actuation means comprises a handle mounted in or on a toilet having a flushing device which handle is capable of moving from a first position of use to a second position of use and vice versa and when moving from said first position of use to said second position of use actuates the flushing device and also moves the lever means from the primary position of use to the secondary position of use.

4. A device as claimed in claim 1 or 3 wherein the mounting means comprises a housing attachable to the surface of a wall of a cistern of the toilet, the wall having the actuation means mounted thereon.

5. A device as claimed in claim 4 wherein the housing is attachable to the external surface of the wall.

6. A device as claimed in claim 4 wherein the housing is attachable to the internal surface of the wall, the cistern having an opening in register with the aperture for permitting the charge of product to exit therethrough.

7. A device as claimed in claim 5 or claim 6 having a decorative diffuser mountable on the external surface of the wall so as to occlude the aperture, the decorative diffuser having one or more apertures for enabling the charge of product to exit therethrough.

8. A device as claimed in claim 7 having an absorbent material locatable between the decorative diffuser and the aperture which material serves to absorb the charge of product and enable a controlled sustained release of product therefrom.

9. A device as claimed in any of claims 1-8 wherein the mounting means includes a housing for the container, the mounting means and the housing having cooperating means for enabling the housing to move from in-use condition, wherein the product release means is in contact with the lever means, to an out of use condition, wherein the container may be removed from the housing.

10. A device as claimed in claim 2 wherein the door is capable of moving from a closed position of use to an open position of use and vice versa and when moving from said closed position of use to the open position of use or vice versa moves the lever means either from the primary position of use to the secondary position of use or from the secondary position of use to the primary position of use.

# Fig.1.

EP 0 512 841 A1

Fig.2.

EP 0 512 841 A1

Fig.3.

Fig.4.

EP 0 512 841 A1

Fig.5.

| European Patent Office | EUROPEAN SEARCH REPORT | Application Number |
| --- | --- | --- |

EP    92 30 4137

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
| --- | --- | --- | --- |
| X | GB-A-974 434 ( RODNEY ALFRED HAWKINS)<br>* claims 1-13 *<br>--- | 1-2,10 | A61L9/14 |
| Y | GB-A-411 055 (JUAN ARTIGAS-ALART CASAS)<br>* figures 1-5 *<br>--- | 1-10 | |
| Y | GB-A-593 454 (CALMIC LIMITED)<br>* claims 1-6 *<br>--- | 1-10 | |
| A | US-A-2 178 888 (STANLEY N. FELDSTEIN)<br>* figures 1-4 *<br>--- | 1-10 | |
| A | GB-A-424 026 (LAVODOR LIMITED)<br>* figure *<br>--- | 1-10 | |
| A | US-A-4 358 860 (DAVID R.CHURCH)<br>--- | | |
| A | DE-A-2 851 139 (TOTORAT)<br><br>----- | | |
| | | | TECHNICAL FIELDS SEARCHED (Int. Cl.5 )<br><br>A61L |

The present search report has been drawn up for all claims

| Place of search | Date of completion of the search | Examiner |
| --- | --- | --- |
| THE HAGUE | 16 JULY 1992 | M.  ESPINOSA |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)